# EUROPEAN PATENT APPLICATION

(11) **EP 1 106 702 A1**
(43) Date of publication of application: **13.06.2001**
(21) Application number: 00126229.4
(22) Date of filing: 30.11.2000
(51) Int. Cl.: C12Q 1/68, C12Q 1/70

(54) **High-throughput screening of compounds using electrospray ionization mass spectrometry (ESI-MS)**

(30) Priority: 08.12.1999 US 169552 P; 07.03.2000 US 521106
(71) Applicant: AGOURON PHARMACEUTICALS, INC., La Jolla, CA 92037-1020 (US)
(72) Inventor: Greig, Michael James, Carlsbad, California 92009 (US); Robinson, Jessica Marie, Carlsbad, California 92009 (US)
(74) Representative: Hofmann, Harald

(57) **Abstract**

A high-throughput screening system employing electrospray ionization mass spectrometry (ESI-MS) to investigate the interactions of between small molecules and polynucleotides and monitor small molecule:polynucleotide complexes, thereby allowing valid mechanistic interactors to be distinguished from false positive signals. ESI-MS may also be used as an early-stage toxicity screening tool.

## Description

This application claims priority from co-pending United States Provisional Application Serial Number 60/169,552, filed December 8, 1999, the contents of which are incorporated by reference herein in their entirety.

### TECHNICAL FIELD AND INDUSTRIAL APPLICATION OF THE INVENTION

The invention generally relates to an assay and associated method for improving the accuracy and specificity of high-throughput screening assays, particularly those associated with biological targets. The assay involves the use of electrospray ionization mass spectrometry (ESI-MS) to investigate the interactions between small molecules and polynucleotides and monitor the formation of small molecule:nucleotide complexes. The assay and method may be used to identify putative carcinogens or to distinguish false positive signals from true positive signals (valid mechanistic interactors) in a pharmaceutical primary screen of test compounds.

### BACKGROUND OF THE INVENTION

Using combinatorial chemistry techniques, wherein a wide variety of small organic molecules are synthesized using combinations of building block reagents, scientists are capable of producing vast numbers of highly complex compounds possessing drug-like features. Likewise, the discovery of numerous therapeutic targets has been accelerated by the Human Genome project and the companion field of bioinformatics, enabling the rapid identification of gene function. In their search for means to thoroughly investigate these newly discovered therapeutic agents and therapeutic targets on an individual basis, pharmaceutical and biotech companies turn to shotgun approaches for assaying the biological activity of large numbers of compounds at once. High-throughput screening (HTS) allows thousands of compounds to be screened against a test assay in a systematic and automated fashion. High-throughput technology has been used to identify viral inhibitors such as those against HCMV and HCV, respectively [Bedard, PJ et al., *Antiviral Res,* **41**(1):35-43 (Feb, 1999); Martell, M et al., J *Clin Microbiol.,* **37**(2):327-32 (Feb, 1999)]. In addition, high-throughput hybridization methods have also been used in the determination of viral titers [Atkinson, EM et al., *Nucleic Acids Res.,* **26**(11):2821-3 (Jun 1, 1998)].

The primary goal of HTS is to identify "lead" compounds, compounds that specifically affect a particular biological target in a desired manner. A typical high-throughput primary screen, assaying a large library of compounds for molecules of biological interest, identifies thousands of "hits". These hits are compounds reacting with the test assay in such a manner as to suggest biological activity. While offering the advantage of testing large numbers of diverse entities in a facile manner, HTS often suffers from the inability to discern true hits, i.e., valid mechanistic inhibitors (or activators) whose interaction with the assay is structure specific, from false positives, i.e., initial hits having non-specific interactivity with the assay components or environment. Additional secondary assays are often required to filter out those compounds lacking biological relevance and to progress initial hits to lead candidate status. For example, scientists use viral screens to identify inhibitors of a particular viral enzyme or catalytic pathway. In these viral assays, a compound's ability to inhibit is often measured by changes in the ratio of double- to single-stranded DNA in the reaction vessel. Artifacts can result if the test compound prevents the DNA from unwinding or otherwise interferes with the components and conditions of the primary assay. For example, a positive signal can result if the compound interacts with the DNA instead of the target enzyme. Follow-up screens are then necessary to determine the compounds true mechanism of action. Current methods utilized to determine oligonucleotide interactions with small compounds include NMR, circular dichroism, gel shift assays, surface plasmon resonance, ATPase assays and the like. Compared to mass spectrometry based assays, these methods require more sample and increased analysis time and yield data that is difficult to interpret. These assays, in this context referred to as "secondary screens", are not only costly and time consuming but they often fail to provide explicit results and details regarding the exact mechanism of enzyme inhibition.

The lack of specificity of the results associated with both primary and secondary screens can be extremely problematic, particularly since the goal of HTS is the identification of potential drug molecules with the desired biological activity in a rapid and stream-lined fashion. Thus, there continues to be a need in the art for fast, reliable analytical techniques having the requisite levels of specificity and accuracy to sort through the libraries of compounds to identify leads for drug development. There is further a need in the art for a mechanism for confirming that specific compounds in the pool of "hits" are in fact "leads", i.e., agents having the specific biological activity with respect to a target.

Herein, a simple and rapid method for identifying artifact-causing, polynucleotide binding test compounds is described, the method being particularly useful in identifying intercalators and other carcinogens. As such compounds are not considered to be useful therapeutics for non-cancer related disorders, they may be removed from the original compound library as well as any future libraries.

### SUMMARY OF INVENTION

The invention relates to an assay or method for measuring the binding between a test compound and a substrate mixture, wherein the mixture components comprise double-stranded polynucleotide and its associated complementary single strands in equilibrium. In one general embodiment, the invention comprises the steps of:
(a) providing a solution comprising at least one test compound and at least one substrate mixture component;
(b) converting the solution to a gaseous spray of ions by electrospray ionization;
(c) transferring the ions in the spray into a mass spectrometer;
(d) monitoring the non-covalent complexes formed between the substrate mixture components and the test compound; and
(e) assessing the results of step (d) to identify those test compounds having specific non-covalent interactivity with the substrate mixture components.

In one preferred embodiment, the invention involves screening a pool of initial hits obtained from a primary high-throughput screen to rapidly differentiate between false positive signals and true mechanistic interactors. The invention involves using electrospray ionization techniques to nebulize a solution containing at least one test compound, the test compound being an initial or primary hit having potential therapeutic utility, and double- and single-stranded polynucleotide substrates which exist in equilibrium, and using mass spectrometry techniques to monitor the formation of non-covalent polynucleotide:test compound complexes to determine if the agent interacts with the substrate. In this manner, one can identify those compounds whose initial reactivity with the primary assay resulted from interaction with the other assay components rather than the specific biological target.

In another preferred embodiment, the high-throughput screen involves a biological target which associates with a polynucleotide. The test compound is screened for its biological activity with respect to the target, either inhibition or activation of the target and the biological process associated therewith. The biological process is preferably a catalytic reaction, more preferably an enzymatic reaction. Other embodiments involve the direct observation of the association of small molecules to strands of messenger RNA or regulatory regions of duplex DNA such as operons, promoters, and the like.

Another object of the invention is to provide an early-stage toxicity screen to uncover polynucleotide-interacting compounds in molecular libraries or subsets thereof. In light of the association of such interacting compounds with carcinogenesis, they are generally considered to be negative leads that should be removed from high-throughput libraries containing potential pharmaceutical lead compounds.

Another objective of the instant invention is to provide an assay and method for the rapid identification of potential carcinogens and cytotoxic agents, such as intercalators and groove binders, in a high-throughput molecular library or subset thereof. In a preferred embodiment, the subset comprises a pool of initial hits identified within the library using a primary assay. Electrospray ionization techniques are used to nebulize a solution comprising at least one test compound and double- and single-stranded polynucleotide substrates and mass spectrometry is used to monitor the formation of non-covalent polynucleotide:test compound complexes. Those compounds that preferentially and non-covalently complex with the double- or single-stranded polynucleotides are referred to as "polynucleotide interacting agents". As discussed below, agents that interact with nucleotides in such a manner tend to be strongly carcinogenic or cytotoxic, and therefore have limited therapeutic potential. Identified polynucleotide interacting agents may be then eliminated from the pool of initial hits or removed from the particular high-throughput library. In addition, when it is desired to eliminate these agents from other molecular libraries when screening for non-cancer therapeutics, ESI-MS can serve as an early-stage toxicity test in a drug development process.

Using the ESI-MS based method disclosed herein, false positives can be easily identified and removed from the pool of primary hits, thereby reducing the overall number of hit compounds requiring further evaluation and testing. Thus, ESI-MS allows one to more rapidly focus in on those biologically and therapeutically significant test compounds, avoiding delay and expense associated with continued research on compounds that later turn out to be inefficient, ineffective or cytotoxic.

### BRIEF DESCRIPTION OF DRAWINGS

Figure 1 shows an ESI mass spectra of known intercalator, distamycin A (DstA), with 10 µM DNA duplex. Graph 1A depicts the spectra at 0.3 µM DstA. Graph 1B depicts the spectra at 1.0 µM DstA. Graph 1C depicts the spectra at 3.3 µM DstA. Graph 1D depicts the spectra at 10.0 µM DstA.

Figure 2 shows ESI mass spectra of known intercalator, ethidium bromide(EtBr), with 10 µM DNA duplex. Graph 2A depicts the spectra at 0.1 µM EtBr. Graph 2B depicts the spectra at 0.33 µM EtBr. Graph 2C depicts the spectra at 1.0 µM EtBr. Graph 2D depicts the spectra at 3.3 µM EtBr. Graph 2E depicts the spectra at 10.0 µM EtBr.

Figure 3 shows ESI mass spectra for known intercalator, proflavin, with DNA duplex. Graph 3A depicts the control spectra, 10 µM DNA duplex. Graph 3B depicts the test spectra, 10 µM proflavin with 10 µM DNA duplex.

Figure 4 illustrates schematic of DNA interactions. Illustration 4A depicts major groove binder, distamycin A. Illustration 4B depicts intercalator, ethidium bromide.

Figure 5 is a schematic of the interior components the Finnigan LCQ ion trap mass spectrometer.

### DETAILED DESCRIPTION OF THE INVENTION AND PREFERRED EMBODIMENTS

An assay using electrospray ionization mass spectrometry (ESI-MS) to was developed to determine the interactions between small molecules and polynucleotide substrates. This solution-based assay can be used to screen double- and single-stranded polynucleotides simultaneously in the presence of individual or multiple compounds, with readout times of less than 5 minutes.

As used herein, the terms "high-throughput screen" or "HTS" refer to an assay involving the exposure of a known (or target) to a group (or library) of unknowns (or test compounds) in an automated fashion, wherein the progression of a reaction associated with the target is assayed for, the results of which correlate to the level of biological activity of a particular test compound. This target-associated reaction often involves binding, such as ligand to receptor or antibody to antigen. In the context of the instant invention, binding includes both complex formation (such as enzyme:substrate, enzyme:inhibitor, or inhibitor:substrate) and hybridization (such as two complementary polynucleotide strands). Biological activity may be measured by other reactions including (but not limited to) cleavage and replication.

A preferred primary HTS assay is a viral screen wherein the percent inhibition of a test compound is based on the amount of duplex polynucleotide unwinding. The degree of hybridization of complementary nucleotide strands is measured against the calculated ratio of double- to single-stranded polynucleotide.

A "target" may be virtually any molecule that reacts with a substrate to yield a product or produce an observable response or catalyze the conversion of substrate to product. For example, a target may be an enzyme, a protein, a polypeptide or the like. Exemplary enzymes include (but are not limited to) kinases, integrases, transferases, helicases, endonucleases, exonucleases, proteases, peptidases, phosphotases, phosphatases, and polymerases. In a preferred embodiment, the biological process is a virus-associated process, more particularly a viral enzymatic reaction. In an especially preferred embodiment, the target is a viral enzyme. The viral enzyme is preferably one associated with a critical step in the virus life cycle, such as replication of viral DNA, RNA, or the viral genome, or integration of the viral genome into the host. Examples of viral enzymes contemplated by the invention include (but are not limited to) HCV helicase, HCV polymerase, HIV integrase, HIV protease, and the like.

The virus making the viral enzyme is preferably one that is causally connected to a pathological condition or disease in a host. The particular virus is not critical and may be any one associated with human, plant or animal pathologies. The virus may be a retrovirus, an adenovirus, a rotavirus, an enterovirus or the like. Examples of virus contemplated by the invention include (but are not limited to) human immunodeficiency virus (HIV), human papilloma virus (HPV), respiratory syncytial virus (RSV), hepatitis A virus (HAV), hepatitis B virus (HBV), hepatitis C virus (HCV), cytomegalovirus (CMV), herpes simplex virus (HSV), herpes zoster virus (HZV), Norwalk virus, Ebola virus, Epstein-Barr virus, Hanta virus, influenza, rhinovirus and the like.

As used herein, the terms "test" and "test compound" refer to an organic compound of unknown biological activity. The test compound may be an oligopeptide, polypeptide, protein, nucleic acid, polynucleotide (e.g., RNA, double- or single-stranded DNA), small molecule, or any mixture thereof. The test compound may be natural or synthetic. The test compound may be a component of a natural product, such as an organic tissue (e.g., hair, skin, kidney, liver tissue, pancreatic tissue, thymus tissue, brain tissue) or a bodily fluid or secretion of an organic tissue (e.g., blood, urine, sputum, semen, sweat, or milk). The test compound may further be a component of cell culture (e.g., bacterial, fungal, yeast) including (but not limited to) culture medium, cellular extract, or culture supernatant. The test compound maybe a molecule or mixture of molecules generated by combinatorial methods.

The test compound is preferably a potential therapeutic, useful in the inhibition or activation of the biological process associated with the biological target. In one preferred embodiment, the process is a catalytic reaction and the biological target is an enzyme.

As used herein, the term "library" refers to a group of different test compounds that can be combined together in a single mixture, divided into several mixtures, or separated into discrete, single-component fractions. The library can be dissolved or suspended in a solvent, buffer, or other carrier or can exist neat.

The term "substrate" refers to a particular polynucleotide sequence recognized by the target -- a polynucleotide sequence that normally reacts with or is catalyzed by a given target molecule. The substrate may comprise double- or single-stranded polynucleotides, including (but not limited to) ribosomal, sense, template, or anti-sense RNA, genomic or copy DNA. In a preferred environment, the substrate comprises double- or single-stranded DNA. In an alternative embodiment, the substrate comprises messenger RNA.

The term "biological screen" refers to an assay involving the manipulation of a biological process. In a preferred embodiment, the biological screen is used to identify candidate inhibitors (or activators) of the biological process, the inhibitors serving as lead compounds for discovery of drug molecules for preventing or treating the pathologic conditions associated with the biological process. In a preferred embodiment, the process is a catalytic reaction associated with a biological enzyme.

A "primary screen" refers to a preliminary assay for biological activity associated with a target compound. In a preferred embodiment, the assay involves the rapid screening of a large test compound library for reactivity in a particular assay (referred to herein as the "primary assay"). The primary assay purports to identify those compounds from the library having specific interactivity and effect on the target. However, primary assays frequently generate a large number of false positive signals, the false positives resulting from non-specific interaction with the assay rather than specific interaction with the target. The pool of compounds generating a positive signal (both true and false positives) in the primary assay are referred to herein as "primary hits".

A "secondary screen" refers to an assay which is used to confirm that the positive signal generated by a "hit" in a primary screen is indeed associated with specific reactivity of the test compound with the target in the primary assay. The assay used in the secondary screen is referred to herein as a "secondary assay" or "confirmatory assay". The secondary assay results in the division of the primary hits into two groups: a first pool of compounds referred to herein as the "false positives" and a second pool of compounds referred to herein as the "true positives". In a preferred embodiment, the interaction assayed for in the secondary screen is the preferential complex formation of test compound:duplex DNA (rather than test compound:single-strand DNA) and the potential carcinogen identified is an intercalator.

The term "viral screen" refers to an assay involving the manipulation of a virus-associated process. In a preferred embodiment, the viral screen is used to identify candidate inhibitors (or activators) of the process, the inhibitors serving as lead compounds in discovery of drug molecules for preventing or treating the pathologic conditions associated with the virus. In a more preferred embodiment, the process is a catalytic reaction associated with a viral enzyme. Alternatively, the viral screen may be a high throughput hybridization assay used in the determination of viral titers.

The terms "electrospray ionization mass spectrometry" and "ESI-MS" refer to a process wherein multiply-charged macromolecular ions are formed directly from solution [Greig et *al., J. Am. Chem. Soc.,* **117**: 10765 (1995); Loo, *Bioconjugate Chem.,* **6**:644-665 (1995)]. This technique provides a rapid, straightforward method for the analysis of small molecule:polynucleotide interactions. ESI-MS allows for the direct observation of small molecule:polynucleotide complexes formed in solution as well as the stoichiometry of the interaction.

The development of electrospray ionization techniques (ESI) for the transformation of proteins and nucleic acids into gas-phase ions has reinvigorated mass spectrometry in the biological sciences. Under proper conditions, gas-phase ions generated using electrospray ionization can retain their solution structures during the desolvation process making it possible to detect and characterize the structure and properties of molecules too large for study via solution techniques such as NMR spectroscopy. The gentle desolvation conditions of the electrospray process preserves non-covalent complexes [Griffey et al., *NATO ASI Ser,* **C510**:117-133 (1998)]. ESI-MS provides a snapshot of solution conditions, facilitating the observation of levels of multiple intermediates along an enzymatic pathway or the formation of complexes, which can not be observed directly in solution. Levels of free and ligated species can be quantitated directly from their relative abundance within the ESI-MS ion envelope. The accurate measurement of molecular mass from multiply-charged ions provides unambiguous information on the stoichiometry of reaction and identity of substrates.

The term "polynucleotide interactor" refers to any compound that specifically interacts with or binds to nucleic acids. The term "polynucleotide" encompasses a variety of double- or single-stranded nucleic acid entities including (but not limited to) ribosomal, sense, template, or anti-sense RNA and genomic or copy DNA. Preferably, the polynucleotide is a deoxyribonucleic acid. Intercalators, major and minor groove binding ligands, and chelators are examples of polynucleotide interactors. Polynucleotide interaction has been closely linked to carcinogenesis. In light of their carcinogenic potential, polynucleotide interactors are deemed negative leads for most non-cancer drug discovery projects.

An "intercalator" is a compound having a crystalline lattice which acts as an electron donor and has "foreign" electron acceptor atoms interspersed or diffused between the planes of the lattice. In other words, an intercalator is a small molecule capable of inserting itself between stacked pairs of bases in polynucleotides. Such intercalation results in mutagenesis, the consequence of which are downstream errors (in transcription, replication, etc.) that frequently lead to neoplastic conditions. Intercalators are considered to be significant carcinogens. Examples of known intercalators include ethidium bromide, proflavin and methyl green (all available from Sigma, St. Louis, MO).

"Groove binders" are a subset of polynucleotide interactors. Groove binders are chemical entities capable of hydrophobic binding to the grooves formed by the helix of double-stranded nucleic acids, such as duplex DNA. [See George et al., *J of Biol. Chem,* **267**(15): 10683-10689 (May 25, 1992) for detailed discussion of DNA intercalators and groove binders]. Examples of known groove binders include distamycin and bis-benzamide (H33258) (available from Sigma, St. Louis, MO).

High-throughput screens (HTSs) generally include a test compound (X) and target (T). HTS involves the screening of a library of test compounds in a test assay to identify compounds which interact with the target or target reaction. The test assay commonly involves direct binding, such as a ligand to a receptor, or antibody to antigen. It is often difficult to directly measure such binding. In these cases, a marker or reporter (M) that is easily measured by direct means is needed as an indirect indicator of binding. For example, when the test assay is an immunosorbent assay, such as an ELISA or RIA, the reporter may be a fluorescent or radioactive compound that is activated upon binding of test compound and target or disappearance of free test compound or target from the reaction environment.

In other cases, the test assay may involve a catalytic reaction, such as action of a target enzyme on a substrate to produce corresponding products. A test compound is screened for ability to interfere with or inhibit the progress of the reaction. While the interaction may also involve binding, the binding is frequently not direct binding of test compound to target. Enzymatic reactions often involve a series of intermediates. A test compound may inhibit any one of the reactions along a catalytic pathway to serve as an inhibitor of a particular target enzyme. A reporter or marker that merely indicates direct binding of target to substrate or test compound to target is insufficient as an indicator of reaction progress or inhibition. Thus, the progress of the reaction must be measured by another means. In certain cases, it is possible to directly measure the formation of product (or lack thereof). However, product production, like binding, is often difficult to measure directly. Therefore, scientists frequently measure a compound's ability to inhibit as a function of the change in the ratio of double- to single-stranded DNA in the reaction vessel. Biological or viral assays are examples of high-throughput screens that are reaction-driven rather than binding-driven.

In both binding- and reaction-driven screens, it frequently is desirable to confirm the validity of the positive results. One must confirm that the hits are indeed associated with the occurrence of the desired reaction and not with a secondary or competing reaction. For example, if one merely tests for binding of test compound as measured by the absence of free test compound, X, in the media, one can not be certain that the test compound indeed reacted with the target. Test compound reacting with markers, reporters, substrates or other reaction component can yield a positive result as well. This is particularly a problem when the assay is a viral screen.

The components of a viral screen include target (T), test compound (X), and one or more substrates (S), which are DNA or RNA polynucleotides. In a preferred embodiment, the target is an enzyme and the test compound is a potential inhibitor or activator of the enzyme. The substrate in the inventive viral screens is a polynucleotide, more preferably a small oligonucleotide. In preferred viral screens, the test assay involves the reaction of the target enzyme with a specific polynucleotide sequence or structure. The polynucleotide may be double-stranded DNA and the reaction is cleavage, unraveling, replication, or the like. In such cases, the reactivity of target enzyme with the polynucleotide substrate is often correlated with the ratio of double- to single-stranded DNA in the reaction vessel. First, a control reaction, involving just target and substrate, is performed to set a standard for relative measurement. Next, one introduces a test compound (or library of test compounds) into the reaction vessel and measures the ratio of double-stranded ("ds") to single-stranded ("ss") DNA. The change in this ratio (ds:ss) is then correlated with the biological activity of the test compound, either inhibition or activation of the target. However, the change in the ratio may be due to artifacts other than inhibition. For example, the test compound may be binding to substrate or intercalating in the nucleotide sequence. Alternatively, the test compound may have the same activity as target (e.g., may itself cleave dsDNA making it appear to be an activator of target enzyme).

The biological screen generally involves the rapid evaluation of a pool of test compounds for inhibition (or activation) of a target. In a preferred embodiment, the target is a biological enzyme involved in the replication of DNA. In this case, the reaction vessel would include at least one test compound, at least one target, and the appropriate substrates corresponding to the target (generally duplex DNA). In addition, the corresponding single strands of the duplex DNA are included to allow one to easily measure the activity of the target, the activity being directly proportional to the degree of unwinding of the DNA duplex. This unwinding measurement is calculated by the change in the ratio of double- to single-stranded DNA in the reaction vessel over the course of the reaction time. If the target is functioning normally, more DNA will be unwound, creating more single strands in the reaction vessel, causing the ratio of double- to single-stranded DNA to increase. When the target is not able to function normally, the double-stranded DNA will remain in duplex form, causing the ratio to remain constant or even decrease. As discussed previously, one may observe a constant ratio and assume that the test compound is inhibiting the biological activity of the target when in fact the test compound is preferentially interacting with or binding to the duplex DNA, preventing the target from catalytically reacting with its substrate.

An example of a biological screen is described by George et al. [*J Biol Chem,* **267**, supra.]. who discuss the effects of a variety of DNA-binding ligands on the unwinding and ATPase activities associated with *E.coli* DNA helicase II. The unwinding and DNA-dependent ATPase activities of helicase II were measured using a spectrum of double- and single-stranded DNA substrates. Examples of substrates utilized include a 71-base pair (bp) M13mp7 partially duplexed DNA substrate, a 245-bp blunt-ended, fully duplexed DNA substrate, and single-stranded poly(dT). George et al. found that the intercalation of mitoxantrone into double-stranded DNA generated a complex that impeded helicase II, resulting in both inhibition of ATP hydrolysis and unwinding activity. Though the George et al. assay involved DNA helicases, the basic experimental procedure and protocol of the assay is the same for other biological enzymes. As noted above, the biological screen described by George et al. yields non-specific results in that it is incapable of discriminating between true hits and false positives.

Electrospray ionization produces highly charged droplets by nebulizing liquids in a strong electrostatic field. ESI generates ions from buffered solutions using a variety of ion source geometries including pneumatically assisted electrospray, micro and nanospray, heated capillary sources, and glass capillaries [Greig et al., *J. Am. Chem. Soc.,* **117**: 10765 (1995)]. ESI specifically involves atomizing a solution with an appropriate nebulizing gas (such as nitrogen) dispensed at an appropriate temperature, pressure, and volumetric flow. ESI provides high sensitivity and transmission efficiency for detection of femptomole quantities using Fourier transform ion cyclotron resonance (FT-ICR), quadrupole ion trap, or conventional quadrupole mass spectrometers.

The particular mass spectrometer utilized is not critical to the invention. Examples of suitable mass spectrometers include quadrupole mass spectrometers, quadrupole ion trap mass spectrometers, and Fourier transform-ion cyclotron resonance (FT-ICR) mass spectrometers. In the examples herein, a Finnigan LCQ Deca ion trap mass spectrometer was used (Thermo-Quest, San Jose, CA). The standard fused silica ESI needle was replaced with a 26 gauge stainless steel needle. Additionally, a proportional gas mixer (Western Enterprises, Westlake, OH) was used to supply a 3:1 mixture of N₂:O₂ as both the sheath and auxiliary gas. The addition of oxygen to the nitrogen allows for higher ESI voltages to be used in negative mode without arcing, providing a steadier electrospray and more efficient ionization of small molecules. The LCQ Deca's capillary temperature was adjusted from 160 °C to 250 °C, depending on the experiment. ESI voltage was set to 3.5 KV for automated analysis; the capillary voltage was set to 15V and the tube lens offset was set to 95 V. The lens and entrance lens settings, two important parameters for keeping non-covalent complexes intact, were set to 35 and 80 V, respectively.

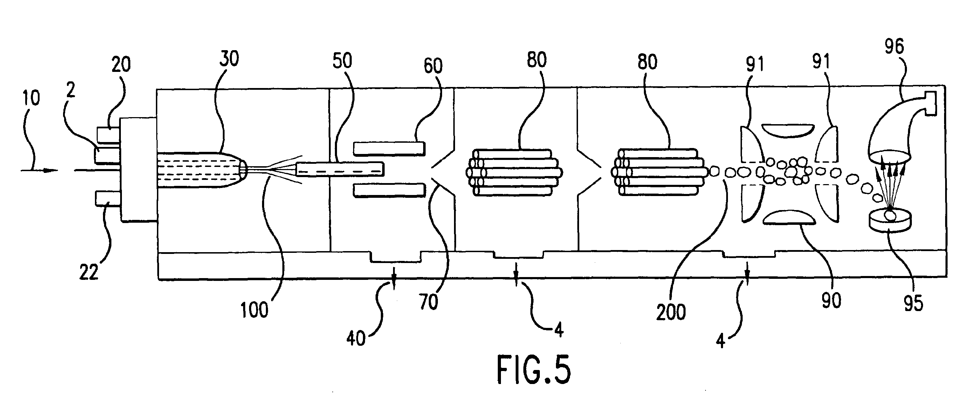

The Q-value for the ion trap should be adjusted so that the trapping is less energetic and gentler on the ions to prevent dissociation of fragile non-covalent interactions. **Figure 5** shows a schematic of the interior components of the Finnigan LCQ. These components are identified by element number in the following discussion.

Aspects of the invention can be automated. For example, the liquid handler may be programmed to automatically take samples from wells of 96-well plates and the like. Such an autosampler can dramatically reduce the overall testing time, allowing a large number of compounds to be screened within a short window of time. The autosampler should be capable of directly injecting the sample at a slow, smooth and constant flow (about 200 nl/min). The sample should not be added to a pre-existing stream (e.g., diluted) or taken as a single plug or bolus The data analysis steps may also be automated. For example, one may utilize an online computer to examine the mass spectrometry results, look for predefined peaks and quantitate the masses and proportions of the various complexes observed. Such equipment is commercially available and standard in the art. In the examples discussed herein, an LC/PAL autosampler (from CTC Analytics AG, Zwingen, Switzerland) was used for automated sample preparation and analysis using a 100 µL syringe. A flow rate of 3 µL/min from the LC/PAL was used to introduce sample into the mass spectrometer.

The sample solution generally includes at least the particular polynucleotide substrates (S) known to interact with the target (X) of interest and preferably at least one test (T) compound. The solution may also include an appropriate buffer. An appropriate buffer is one capable of improving ion abundance, such as imidazole [Greig et al., *Rapid Commun Mass Spectrom,* **9**:97-102 (1995)]. The minimum concentration needed to generate a readable signal can vary from one instrument to the next. In order to keep the number of variables to a minimum, it is preferable that the ESI-MS high-throughput secondary screen utilizes substrate and test concentration ranges that are identical to those utilized in the primary screen. As efficient sampling and transport of ions into the mass spectrometer is also desirable, it is preferable to automate the liquid sampling as described below. All liquid connections should be both water- and air-tight.

Referring to the schematic shown in **Figure 5**, sample solution is infused into the apparatus via the analyte-in port (10). Nebulizing gas is infused via the sheath gas port (20). In some cases, a "liquid sheath", or a layer of flowing liquid in conjunction with a chromatographic/electrophoretic effluent, is used to augment the performance of the separation-ESI-MS combination. Such a liquid sheath solution may be infused via the sheath liquid port (21). Additional gas may be infused via auxiliary gas port (22) to focus the nebulized droplets into a stream-lined spray. On the Finnigan LCQ apparatus shown in **Figure 5**, the spray is dried during transport through a heated metal or glass capillary (50) and/or lens housing (60). Drying temperatures typically range from 160 °C to 250 °C. An alternative approach that is available on other instruments involves the flow of a drying gas (generally N₂) with the nebulizing gas. The charged droplets drift against a countercurrent flow of dry gas directed through an axial plenum toward the sampling aperture (10). A curtain of nitrogen, which is generally disposed about the periphery of the capillary tube (50), serves as a drying agent, to exclude large droplets and particles, to decluster ions. Although the countercurrent drying gas flow is not essential, best results may be achieved if the two drying processes are combined. As the dried ions pass through the orifice (ESI needle assembly 30) into the higher-vacuum region, they are accelerated through collision chambers (100, 200) where they are further declustered by energetic collisions with background gas. Vacuum and acceleration are generally achieved via a series of pumps (mechanical pump 40, turbo pumps 41).

Slit-like skimmers (70) are placed in the ion path to define the positions and energies of the ions that strike the detector (electron multiplier 96). The skimmers primarily function as a pressure gradient, facilitating the gradual reduction in pressure from atmospheric to vacuum. Additional electrostatic lenses are preferably used to shape and deflect the ion beam to optimize peak shape and maximize ion beam transmission from the source to the detector. In the Finnigan LCQ, two octapole focusing lenses (80) are used to simplify the ion optical design. The ion beam is then accelerated towards an electrode, preferably a ring electrode (90). The ring electrode (90) generates an electric field that exerts a force perpendicular to the ion motion to deflect ions according to their kinetic energy. The excited ions are then ejected from the electrode and converted and counted by conversion dynode (95) and electron multiplier (96), respectively.

The spectrum of the sample is obtained via infusion of an aliquot of solution at a flow ranging from 5 µL/min to 200 nL/min, more preferably about 200 nL/min. The solution is flowed through the electrospray or microspray source interface (30) of a mass spectrometer. The solution may be a water-IPA, water-methanol or water-acetonitrile mixtures and may contain other additives such as ammonium acetate, acetic acid or formic acid to buffer the solution. The solution may be infused to the source from syringe via a syringe pump or from a liquid chromatography column via a liquid chromatographic pump. The pump delivers a controlled flow of liquid.

The ESI mass spectrum generally involves a bell-shaped distribution of charge states in which adjacent peaks differ by one charge. A feature of ESI-MS for most macromolecules is that the average charge state increases in an approximately linear fashion with molecular weight. The sample generates an envelope of ions that uniquely identifies the components of the sample, acting as a fingerprint for that particular sample, to which other samples and controls may be compared. Spectra are generally obtained as the averaged sum of 15 scans from m/z 1000 to 3000 at a scan rate of 32 s/scan. The raw data are standardly processed with a 0.5 Da Gaussian filter prior to display. The size and material of the microspray needle may vary with particular sample.

The spectrometer may be set in either negative or positive ionization mode. The polarity of the mass spectrometer may be varied from positive to negative to match the components of the same one wishes to identify. As polynucleotides have an overall net negative charge, interactions therewith are studied with the spectrometer in the negative mode. Likewise, as most proteins are fairly easy to protonate, they are generally studied with the spectrometer in the positive mode. For positive ion ESI, a voltage of about 4-6kV is typically applied. In negative ion mode, an electron scavenger such as oxygen or sulfur hexafluoride or air is used to inhibit corona discharge at the capillary tip. The examples described herein began with the spectrometer in the negative mode. However, in those instances where no signal for the bound ligand is detected in the negative mode, one can switch the machine to the positive mode to assay for the presence of the unbound ligand.

The optimum observation of complexes involves optimization to instrument parameters including sprayer position, countercurrent gas flow, capillary temperature, and skimmer and lens potentials. It also involves optimization of macromolecule and ligand concentrations, buffer type and concentration, and content of organic solvent used to stabilize the electrospray. Parameters such as gas used, flow rate, voltage, temperature, calibration criteria, and the like will vary from one instrument to the next. The optimization process is well within the purview of one of skilled in the art. Experimental protocols for optimizing certain parameters, such as capillary temperature and organic solvent, are described in the Examples section below.

It is important to note that the degree of tightness of binding correlates to the degree of interaction. Weak binding to the polynucleotide substrate indicates that the test compound may indeed have been binding to the target and therefore can not be ruled out of the pool of hits as a false positive or polynucleotide interactor. To ensure the accuracy of the characterization of hits, one should look for the presence of strong non-covalent bonding. One should distinguish those that are binding solely due to electrostatic interactions or other non-specific reaction driven by charge differential or the like from those whose interactions are dictated by specific structure. One can distinguish weak from tight binding by comparing the bound (test:substrate) peak to the duplex peak. Tight binding is characterized by a bound peak area of at least 15-20% of the duplex peak area. Weak binding is characterized by a bound peak area of less than 10% of the duplex peak area.

Collision Induced Dissociation ("CID") may be used to measure the specificity and strength of a particular interaction. Collision chambers can be provided in the first field-free region (100), generally located just after the ion source (30), and second field-free region (200), generally located just after the octapole and before the ring electrode (90). These chambers are used to induce ions to fragment in CID experiments.

CID may be increased to such level wherein the single-strand DNA is fragmented while the duplex:small molecule complex remains unperturbed. Also, by increasing salt concentration in the test solution, one can affect the electrostatic binding, resulting in the formation of only those bonds that are due to hydrophobic structural or stacking interactions. By monitoring the effect of salt on the amount bound versus unbound complex, one can distinguish non-specific electrostatic interactions from structure-specific non-covalent interactions. Drastic changes in binding upon the addition of salt to the solution generally correlates with the presence of electrostatic interactions. Thus, as part of the optimization of solution conditions, one may wish to adjust the buffer strength to screen out non-specific interactions. For instance, in the examples below, the concentration of the ammonium acetate buffer was adjusted. As the salt concentration was increased from 10 µM to 50 mM, the binding affinity of ethidium bromide, a known intercalator did not change. However, the binding of a positively charge molecule decreased as competition for ionic sites increased. In addition, the CID source was manipulated as discussed above.

The following examples are provided for illustration purposes.

### EXAMPLES

### Optimization of Experimental Parameters:

As mentioned above, the optimum observation of complexes involves optimization to instrument parameters and experimental conditions. Regarding the instrument capillary temperature, experiments were conducted to determine the optimum temperature for most efficient transfer of sample and maintenance of intact duplex into the ion trap. Using the 6:7 duplex described below, the capillary temperature was adjusted from 150° C to 250° C. The over-abundance and ratio of ss to ds DNA was then monitored. Under the experimental conditions herein {e.g., a flow rate of 1-3 µL/minute with 10 µM duplex in 2:1 water:IPA, 50 mM ammonium acetate}, the temperature of 185° C was found to be optimal, both in terms of sensitivity and duplex stability. Lower temperatures resulted in excess ammonia adducts while higher temperature resulted in increased dissociation of the duplex.

Regarding the organic solvent used to stabilize the electrospray, methanol (MeOH) and iso-propyl alcohol (IPA) are the organic additives of choice for use in the assay herein. However, since DMSO is widely used as a bulk solvent for many compounds in combinatorial chemistry and most of the concentrated samples received for analysis were in DMSO solutions, the limits of DMSO concentrations acceptable for the assay needed to be tested. Test solutions were prepared as stated below in the Examples section [e.g., 10µM duplex; 10 mM ammonium acetate with the addition of 0.5 to 10.0% DMSO in 0.5% v/v increments]. It was found that as little as 1 % DMSO caused over 50% disruption of the duplex. This disruption was identical with and occurred without the presence of either ethidium bromide or proflavin. At 10% DMSO, the duplex was 100% dissociated into its respective single strands. Because the assay herein is meant for high-throughput, the DMSO content was allowed to be as high as 1%, and the dissociation of some of the duplex was acceptable as lone as sufficient signal was obtained to determine binding. The above experiment was repeated with MeOH and IPA, keeping ammonium acetate concentration at 10 mM throughout, and it was found that the duplex was stable in up to 50% of either of these organic modifiers in water.

Regarding buffer concentration, the concentrations of ammonium acetate were varied to determine the effects of ionic strength on ligand binding. Mixtures of 10 µM 6:7 duplex and 10 µM sample were observed with ammonium acetate concentrations varying from 10 mM to 250 mM. Of the intercalators, proflavin tolerated over 100 mM ammonium acetate with minimal shifting in binding. However, ethidium bromide and methyl green had almost 70% reduction in binding to DNA at only 33 mM ammonium acetate. The reduction in binding of ethidium bromide and methyl green at high salt concentrations is evidence of the electrostatic nature of the first step in binding in the intercalation of these compounds. Proflavin, which is neutral at the test pH, should be less affected by an increase in competing counterions. Of the minor groove binders, the distamycin A:DNA complex was stable up to 100 mM ammonium acetate and was still observed (though at much reduced levels) with 500 mM ammonium acetate in solution. H33258 binding, on the other hand, showed an appreciable reduction in binding in 33 mM ammonium acetate; in fact, the relative amounts of the 1:1 H33258:DNA complex compared with the 2:1 H33258:DNA complex shifted at 33 mM ammonium acetate. This suggests that the binding of H33258 to ds DNA is different than the cooperative nature of the binding of distamycin A.

### Sample Preparation and Analysis:

The general protocol for preparing the samples utilized in the present invention is as follows. Complementary single-stranded oligonucleotides are desalted, preferably via ammonium acetate precipitation. This DNA desalting procedure involves the following steps. First, the oligonucleotide is dissolved in approximately 100 µL of water. Next, about 500 µL of 10 M ammonium acetate is added. The solution is mixed and allowed to rest for about 5 minutes. From about 600 to about 100 µL of cold ethanol (EtOH) is added and the solution is set in dry ice for about 25 minutes. The solution is then centrifuged for about 20 minutes. The supernatant is removed and saved and the preceding steps are repeated once. Subsequently, 1 ml of a 90:10 solution of EtOH and water are added and the solution is centrifuged for 5 minutes. This step is then repeated twice. The solution is then dried and rehydrated. Finally, OD readings are taken at 260 nanometers to ensure a 1:500 dilution.

The complementary oligonucleotide strands and corresponding oligonucleotide duplex are not critical to the invention. The duplex may comprise a DNA:DNA duplex, an RNA:RNA duplex or a DNA:RNA duplex. The DNA strands utilized herein are described in detail below. Once desalted, the complementary oligonucleotide strands are added in equimolar mixture in 50 mM ammonium acetate to make a 50 pM duplex stock solution. The duplex stock solution is placed in a 60° C water bath for 10 minutes and then slowly cooled to room temperature. Test solutions are made by adding test compound (dissolved in methanol (MeOH)) to stock solution in a 2:1 water:isopropyl alcohol. Final concentrations of test compound and duplex are preferably 10 µM each. For example, 20 µL of the stock duplex solution is added to 80 µL of a 2:1 solution of water and IPA for a final volume of 100 µL and a final concentration of 10 µM duplex. The test compound is then added to the solution for a final concentration of 10 µM as well. One may subsequently dilute the test solution to test a range of test compound concentrations, from as low as 0.3 µM up to 10 µM.

Once the appropriate samples and solutions are prepared, one may proceed with the ESI-MS procedure. The general protocol for manual analysis is as follows. First, a 50 µL sample of test solution (comprised of about 30 picomoles of test compound) is drawn into a 100 µL syringe and infused into an LCQ Decca ion trap mass spectrometer using ESI-MS source. The sample is directly infused at a rate of about 1.5 µL/min over a period of approximately 2 minutes. The spectra is then analyzed for the presence of bound test ligand. If the test ligand does bind, the sample is re-run at lower concentrations, from about 0.3 µM up to 1.0 µM. In order to ensure statistical accuracy, data are preferably summed, saved, and analyzed over a large numbers of scans, preferably 30 to 50 scans, more preferably 40 scans.

As discussed above, an autosampler, such as the LC/PAL system, can be used to automate sample preparation and analysis. Parameter commands specific to the particular experiment are first entered into the LC/PAL system. All subsequent mixing and solution transfers may then performed by the LC/PAL without user intervention. The cycle time for sample preparation is approximately 2.5 minutes/sample. Much of this time was used for washing the syringe to prevent sample carryover. 250mM samples of ligand are supplied in either 96 well plates or standard HPLC vials. 10 µL of the 50 µM duplex stock solution, 15 µL of isopropanol, and 25 µL of water are mixed in a plastic HPLC vial with a 150 µL limited volume insert. Next, 2 µL of the ligand solution is added and mixed. Although most of the ligands assayed came to equilibrium within 60 seconds, the solution remains at room temperature for 30-90 minutes prior to analysis so as to ensure that the solution components have achieved equilibrium. Equilibrium in this context is defined as no change in the ratio of bound to unbound ions over time.

The general protocol for automated analysis is as follows. First, a 31 µL sample of test solution is aspirated into the LC/PAL's 100 µL syringe. Next, 20 µL (the system's dead volume) is injected into the tubing to the ESI-MS source at 20 pL/min. The flow rate is then reduced to 3 µL/min for MS analysis. For the first minute, scans are summed alternatively positive and negative mode from 80-800 m/z (a mass range that encompasses all of the test ligands) then changes to 1200-2000 m/z for an additional 1.5 minutes. The total cycle time, including extensive washing, is about 6.5 minutes/sample. For those samples where the mass and purity of the test compound is known, data analysis was automated. For other test compounds, data analysis was completed by hand. A blank and the duplex of interest with 2 µL of solvent added were included with each set of determinations. Under the conditions set forth herein, a 10 µM DNA duplex in 2:1 10 mM ammonium acetate in water:IPA mixture yields a spectrum with over 95% of the DNA in the duplex state.

### Oligonucleotide and Test Samples:

The oligonucleotides utilized in the examples herein were known to interact with a particular target(s) of interest. They were provided according to our specifications from TriLink (La Jolla, CA). In the experiments described below, a 21-mer oligonucleotide strand having the following sequence was used: 300-6: GTA TGA CGC TAG TGT ATG ACC (MW=6,462.00) An oligonucleotide strand complimentary to 300-6 was then synthesized, the sequence set forth below: 300-7: GGT CAT ACA CTA GCG TCA TAC (MW=6391.00) These two oligos create a duplex (MW=12,853.00) that does not have any overhanging single-strand segments. When the duplex solution was analyzed by ESI-MS, it was possible to see at least three charge stages of the duplex as well as some charge states of the two single strands. A mixture of such oligonucleotides makes it possible to test for binding to duplex and single strand in the same experiment. The 6:7 duplex stock solution was made using 50 µM of single strand 300-6, 50 µM of single-strand 300-7, and 50 mM ammonium acetate solution (NH₄Ac in water). The solution was diluted to a final concentration of 10 µM duplex and 10 mM NH₄Ac. The ammonium acetate was added to enhance and ensure duplex formation. The mixture was heated at 60 ° C for about 10 minutes and then slowly cooled to 25°C (or room temperature) over a period of about 30 minutes. As stated above, this time ensures that the solution components have achieved equilibrium. For insertion into the spectrometer, duplex spray solutions were made using a mixture of 2:1 stock:IPA (60 µl of stock, 30 µl of IPA).

All test compound:substrate preparations were diluted with a solution of 2:1 H20:IPA. The assay utilized a 100 µL sample solution comprised of 10 µL of 100 µM test compound, 20 µL of 50µM duplex stock (10 µM duplex, 10 mM NH4Ac), 46 µL of distilled water, and 24 µL IPA. This initial solution composition is the preferred embodiment. Unless specifically noted otherwise, the data presented in the experimental section utilize such a solution composition, wherein the test compound concentration is 10 µM, the oligonucleotide concentration is 10 µM, and the oligonucleotide is the 6:7 duplex.

Subsequently, various dilutions and new solutions were prepared to test the effect of the change in test compound concentration (from as low as 0.1 µM to as high as 100 µM). The concentration of duplex was held constant at 10 µM.

In the examples below, several known intercalators (proflavin, ethidium bromide, methyl green) and minor and major groove binders (distamycin A, H33258) were tested. The inventive method may also be used to screen a series of primary hits isolated from various compound libraries to identify false positives. The false positives are those that interact with the substrate rather than the target and are generally identified as polynucleotide interactors.

The formation of small molecule:oligonucleotide complexes was monitored in a "Yes/No" fashion at specific ligand concentrations. Some of the properties of the interactions were also investigated. By altering the ionic strength of the buffer, it was possible to differentiate between non-specific electrostatic interactions and more specific interactions. Dissociation constants were calculated for various complexes by titrating the ligand and measuring relative peak intensities of bound and unbound duplexes. Source CID and collisions in an ion trap were used to further characterize these interactions. Differences in the fragmentation patterns of duplex with and without ethidium bromide are discussed.

### Part A -Intercalators and Groove Binders:

### Example 1A - Distamycin A

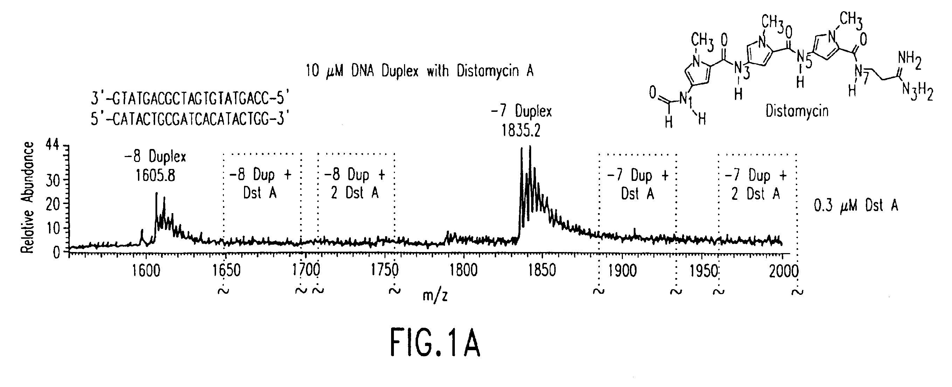

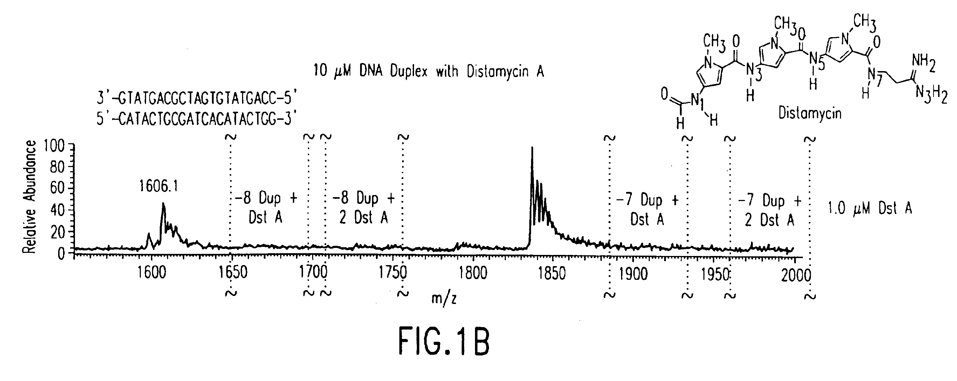

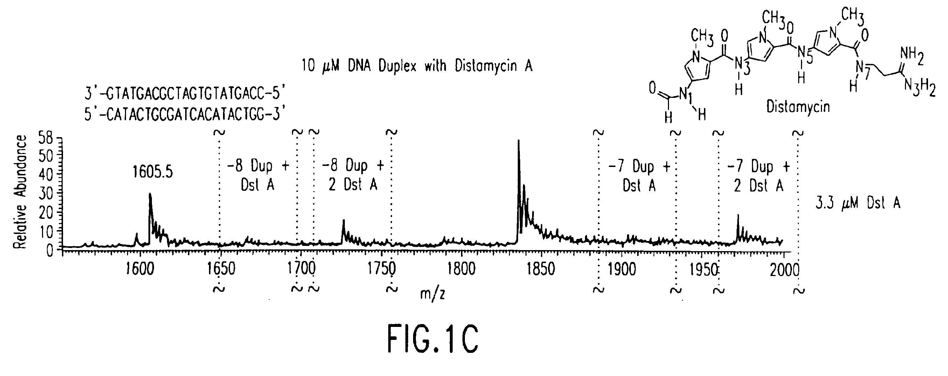

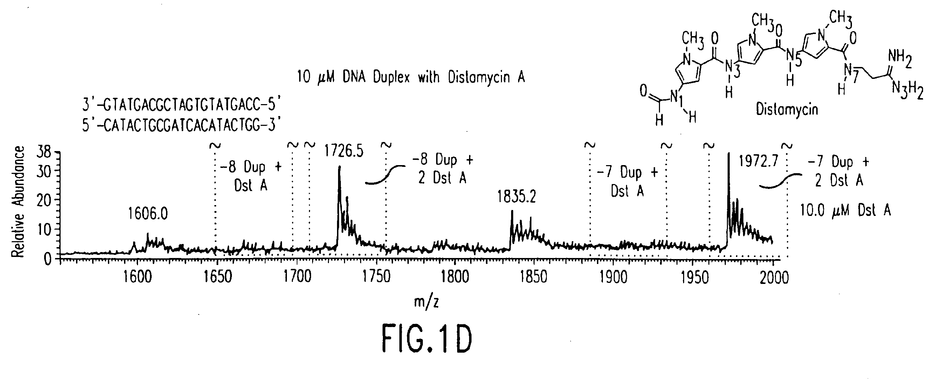

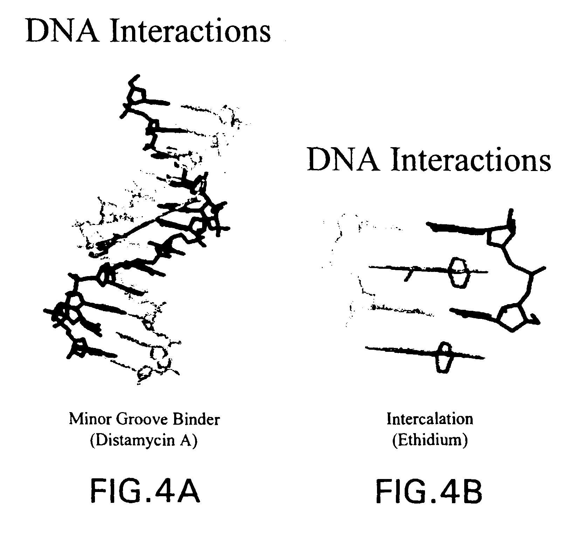

Distamycin A was obtained from Sigma. Our mass spectrometry studies confirm the formation of a 2:1 DstA:DNA complex, although the ratio of bound species appears to be dependent on the oligonucleotides used. The groove binding activity of distamycin is shown in **Figure 4, Graph 4A**. The effect of the distamycin A on the reaction scheme was tested, mixing 10 µM control duplex (6:7) with 0.1 to 10 µM of distamycin A. The results for the assay shown in **Figure 1, Graph 1D** (10 µM of distamycin A) are shown in Table 1 below. The ESI mass spectra for the concentrations of distamycin A (0.3, 1.0, 3.3, and 10 µM) with duplex 6:7 are shown in **Figure 1, Graphs 1A-1D,** respectively.

### Example 2A - H33258

H33258 (bis-benzamide) was obtained from Sigma. The groove binding activity of H33258 was tested, mixing 10 µM control duplex (6:7) with 0.1 to 10 µM of H33258. A 2:1 H33258:duplex ratio was present at higher concentrations (>3.3 µM); at lower concentrations, only the 1:1 mixture was observed. In addition, as the concentration of ligand was increased, the ligand bound to ss DNA was observed in increasing quantities.

### Example 3A - Ethidium Bromide

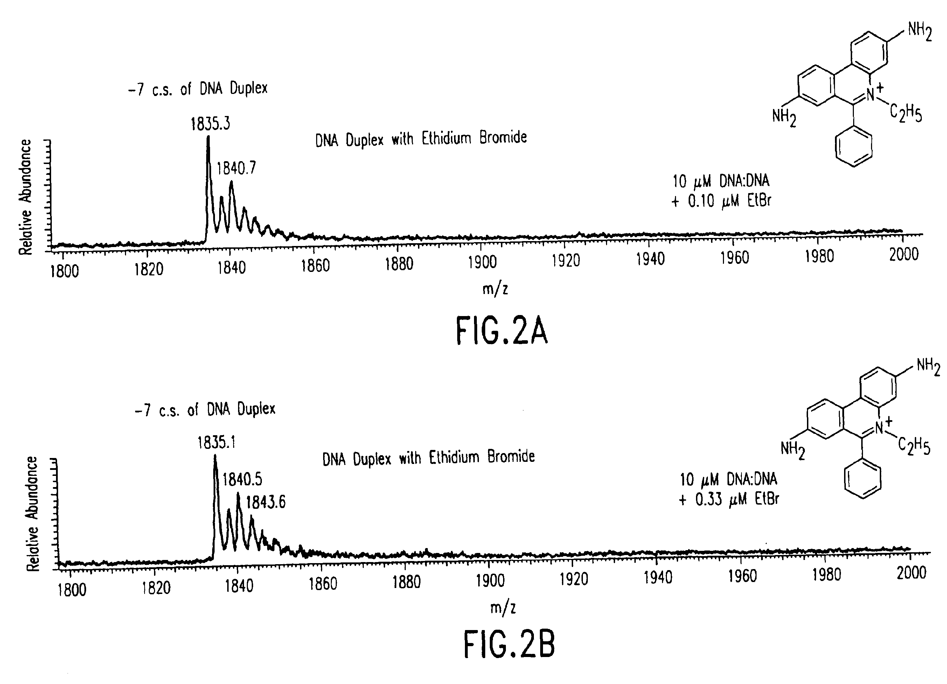

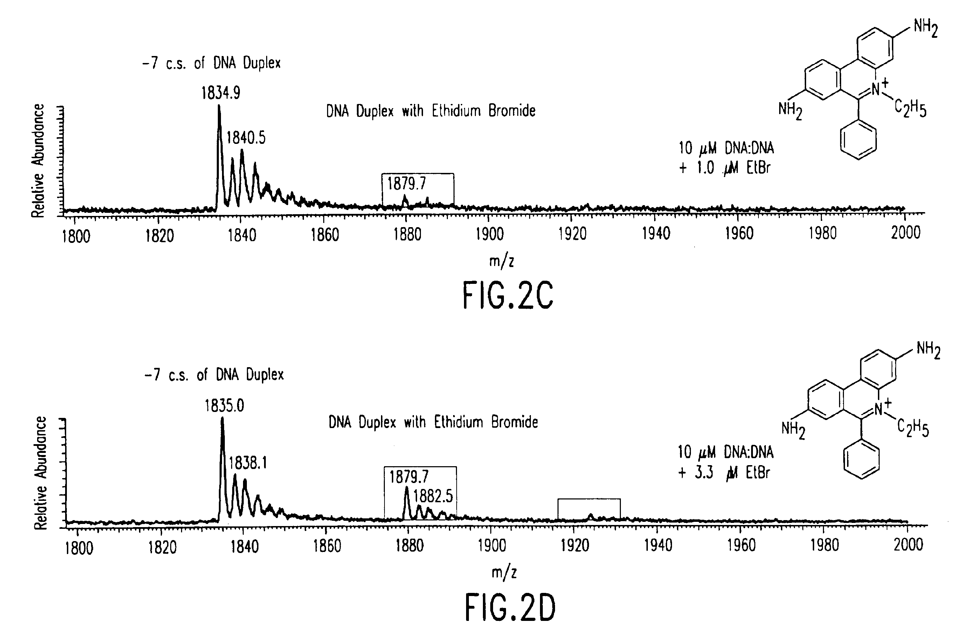

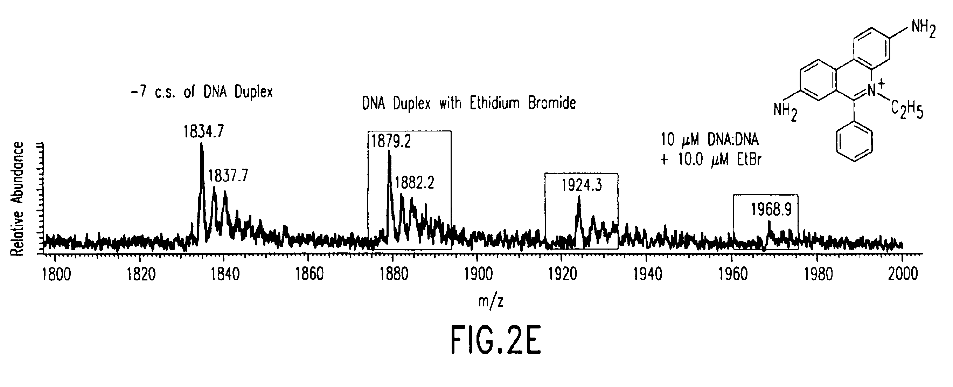

The intercalating activity of ethidium bromide is shown in **Figure 4, Graph 4B.** Ethidium bromide was obtained from Sigma. In the example herein, the control duplex (6:7) was mixed for a final concentration of 10 µM with EtBr for a final concentration of 10µM in 10 mM ammonium acetate and analyzed by ESI-MS. Next, solutions with different component concentrations were analyzed, using varying concentrations of EtBr (0.1, 0.3, 1, 3.3, 10, and 33µM) and ammonium acetate (10, 50 µM). The duplex concentration was held constant at 10 µM. Initial binding was observed at 3 µM EtBr. As the concentration of intercalator was increased, it was found to bind to multiple sites on the duplex. No binding to single strand was observed. when the duplex:ethidium bromide complex was dissociated, fragments included the individual single strands and the single strands with EtBr adducts. See Table 1 below for details of the ethidium bromide testing. The ESI mass spectra for the concentrations of EtBr (0.10, 0.33, 1.0, 3.3, 10.0 µM) with duplex 6:7 are shown in **Figure 2, Graphs 2A-2E**, respectively.

### Example 4A - Proflavin

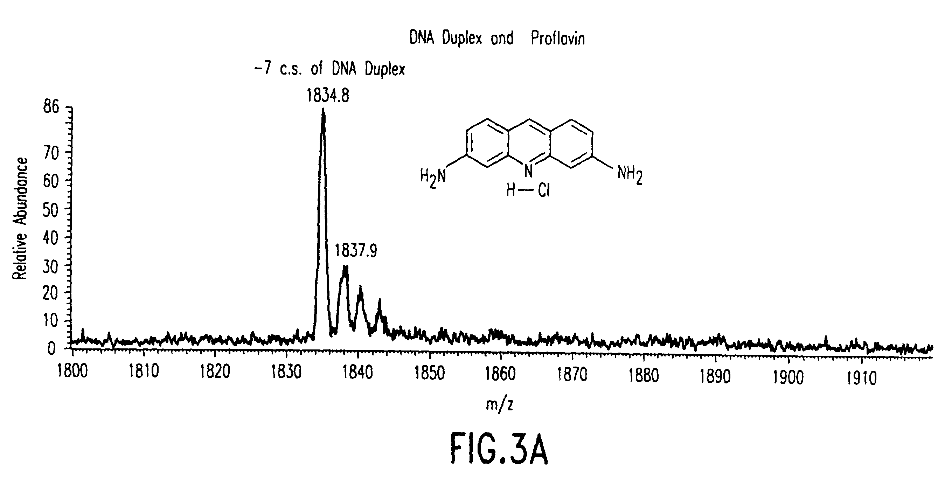

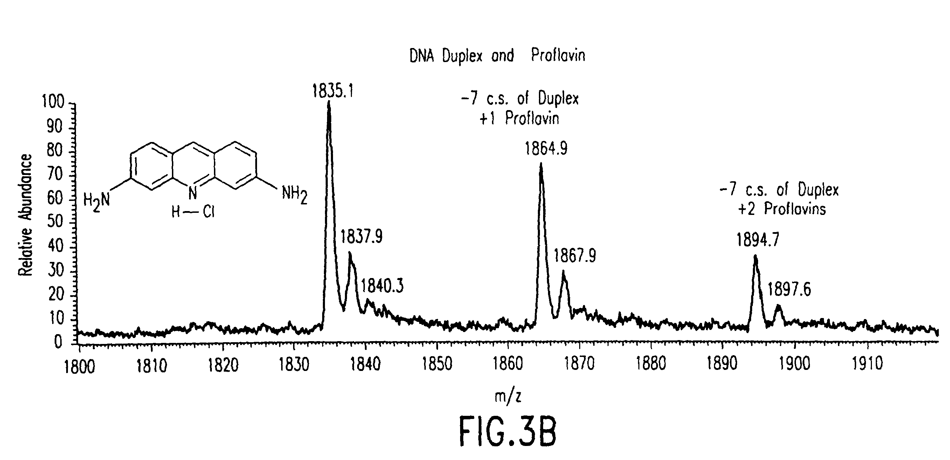

Another known intercalator was tested in the system herein. The proflavin was obtained from Sigma. The 100 µL total volume spray solution comprising 10µL of proflavin (final concentrations ranging from 1 to 10 µM), 60 µL of duplex stock solution (final concentrations ranging from 1 µM to 10 µM), and 30 µL of IPA was prepared. Studies were performed on the FT-ICR mass spectrometer. When using the 10 µM duplex and 10 µM proflavin, binding between intercalator and duplex was observed. Data for the proflavin with 6:7 DNA duplex are shown in Table 1 below and **Figure 3, Graphs 3B**. Data for the DNA duplex alone are shown in **Figure 3, Graph 3A.Table 1**:

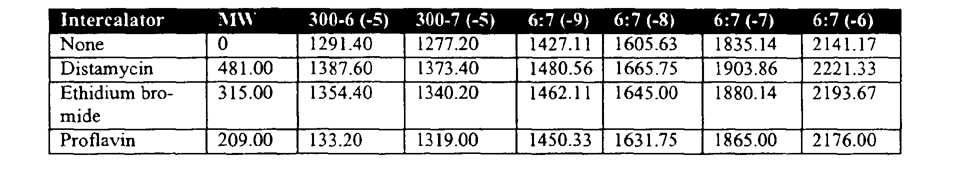

### Example 5A - Methyl Green

The intercalator, methyl green (obtained from Sigma), was tested in the system herein. Methyl green was observed to display similar binding characteristics with the other intercalators. The results obtained show that the relative binding affinity of these intercalators to 6:7 duplex in 10 mM ammonium acetate is methyl green > ethidium bromide > proflavin.

### Part B - The Biological Screens:

### Example 1B - HIV integrase

The human immunodeficiency virus (HIV) integrase (Int) protein mediates an essential step in the retroviral lifecycle, the integration of viral DNA into human DNA [Lutzke et al., *Nucleic Acids Res.* **22**(20):4125-31 (10/11/1994)]. HIV integrase consists of three domains, the structures of which have been individually determined by X-ray crystallography or NMR spectroscopy. The core domain, spanning residues 50-212, is responsible for the catalytic activity of the enzyme. The small N-terminal domain folds into a dimeric helix-turn-helix structure, which is stabilized by the coordination of zinc with conserved His and Cys residues. The function of this domain is unclear; however, it is required for integration activity and enhances tetramerization in the context of the full-length integrase. The C-terminal domain, which has an SH3-like fold, is involved in DNA binding. [Esposito et al., *Adv Virus Res.,* **52**:319-33 (1999)].

HIV integrase unravels duplex DNA to perpetuate HIV infection. Therefore, compounds that inhibit the activity of HIV integrase may be able to prevent the replication and transmission of the virus. Recognition and interaction of the HIV integrase with double-stranded DNA is critical in standard-transfer assays used to identify inhibitors of that enzyme [Hazuda D.J. et al., *Nucl. Acids. Res.,* **22**:1121-1122 (1994)].

Potential hits are identified from an original library of compounds. These hits are then screened to determine if they are true mechanistic inhibitors of the enzyme. Test compounds are prepared as described above, in a solution of 2:1 H₂O: IPA with 10 µM test compound with 10 µM duplex. Test compound can be screened at various concentrations, preferably over a range of about 0.10 up to 10 µM. The various samples can be run on either the Finnigan LCQ ion trap mass spectrometer or the Bruker Fourier transform ion cyclotron resonance (FT-ICR) mass spectrometer. The latter is more sensitive.

By comparing the change in molecular weight peaks observed at a particular charge state, one
can identify if a test compound is binding to the substrates, either double- or single-stranded. Specifically, one assays the test compound peaks, looking for a specific increase in molecular weight as compared to the control peaks, the increase corresponding to mass of unknown divided by charge (m/z). At lower charge states, the increase in molecular weights is greater, the shift in the peaks being more substantial. For example, when testing, a compound having a molecular weight of 311.40, in the negative mode at -9, one would expect an increase in mass of 311.40/9 or 34.6.

### Example 2B - HCV Helicase

Helicases are nucleotide triphosphate (NTP)-dependent enzymes responsible for unwinding duplex DNA and RNA during genomic replication [Yao N et al., *Nat Struct Biol,* **4**(6):463-7 (1997)]. The RNA helicase of the Hepatitis C virus (HCV helicase) is an attractive target for antiviral therapies [Di Bisceglie AM, *Am J Med,* **107**(6B):45S-48S (Dec. 27, 1999)]. Using high-throughput technology, scientists can rapidly search for new antagonists and agonists, inhibitors and activators of HCV helicase. Some HCV helicase assays use the ratio of ss DNA to ds DNA as a reporter. Generally, one oligonucleotide strand is covalently bound to a test well while the other is fluorescent- or radio-labeled. After incubation with the HCV helicase enzyme, duplex DNA, and test ligand for 10 to 20 minutes, the well is rinsed and the fluorescence or radioactivity is measured [Eamshaw D.L. et al., *Jrnl. of Biomol. Screening,* **4**:239-248 (1999)].

The primary assay described above can fail to screen out "negative" leads, such as potential carcinogens and intercalating compounds. Using the ESI-MS technique described herein, primary hits from an HCV helicase based primary assay can be tested for the presence of polynucleotide interacting compounds, particularly compounds that bind to duplex polynucleotide.

### Example 3B - RNAseH

Ribonuclease hybrid (RNAseH) is an endogenous enzyme involved in the process of DNA transcription; RNAseH specifically degrades the RNA strand of an RNA:DNA hybrid [Miller H.I. et al., *Proc Soc Exp Biol Med,* **148**(1):151-6 (1975); Horiuchi T, *Tanpakushitsu Kakusan Koso,* **31**(2):132-43 (1986); Garces J. et al., *Proc R Soc Lond B Biol Sci,* **243**(1308):235-9 (1991)]. Using high-throughput technology, scientists can rapidly search for new antagonists and agonists, inhibitors and activators of RNAseH. One assay for finding inhibitors of RNAse H uses a non-radioactive labeled DNA:RNA heteroduplex as the substrate in the assay [Rychetsky P. et al., *Analytical Biochemistry,* **239**:113-115 (1994)]. DNA:RNA hybrids are A-form helices in solution. Although several interacting ligands only bind to the B-form of DNA, some have been known to associate with these heteroduplexes. This association can induce a transformation from the A-form to the B-form. As in previous examples, the interaction with the oligonucleotides complex by the test ligands can produce a false positive result

The primary assay described above can fail to screen out "negative" leads, such as potential carcinogens and intercalating compounds. Using the ESI-MS technique described herein, primary hits from an RNAseH based primary assay can be tested for the presence of polynucleotide interacting compounds, particularly compounds that bind to duplex polynucleotide.

### Example 4B - CRF

Corticotrophin-releasing factor (CRF) is an endogenous 41-amino acid neuroendocrine peptide involved in a wide ranging series of systems ranging from memory and learning to the coordination of the body's overall response to stress to initiation of labor (known as the placental clock) [Wang, HL, *Eur J Neurosci.,* **10**(11):3428-37 (Nov, 1998); Keller et al., *JMed Chem,* **42**(13):2351-7 (Jul 1, 1999)]. In addition, CRF has been reported to substantial antineoplastic effects, able to both inhibit proliferation, induce differentiation, and decrease vascular permeability in tumor cells. CRF purportedly operates by a mechanism involving the activation of nitric oxide synthase (NOS) and L-arginine-NO pathway [Tjuvajev, *In Vivo,* **12**(1): 1-10 (Feb, 1998)].

Using high-throughput technology, scientists can rapidly search for new antagonists and agonists, inhibitors and activators, of CRF. However, the primary assays fail to screen out "negative" leads, such as potential carcinogens and intercalating compounds. Using the ESI-MS technique described herein, primary hits from a CRF based primary assay can be tested for the presence of polynucleotide interacting compounds, particularly compounds that bind to duplex polynucleotide.

### Example 5B - GnRH

Gonadotropin releasing hormone (GnRH), also known as leutenizing hormone releasing hormone (LH-RH), plays a central role in the biology of reproduction and sexual development. Antagonists and agonists find utility as medicaments for diseases or conditions mediated by modulation of the pituitary-gonadal axis. For example, GnRH agonists have been used to reduce testosterone production, thereby reducing prostate volume in benign prostatic hyperplasia (BPH) and slowing tumor growth in prostate cancer [Lovas et al., *J Pept Res* **52**(5): 384-9 (Nov, 1998)]. These compounds have also been used to treat breast and ovarian cancer. GnRH antagonists have been described in the treatment of endometriosis, uterine myoma, ovarian and mammary cystic diseases, and amenorrhea, for example.

Using high-throughput technology, scientists can rapidly search for new antagonists and agonists, inhibitors and activators, of GnRH. However, the primary assays fail to screen out "negative" leads, such as potential carcinogens and intercalating compounds. Using the ESI-MS technique described herein, primary hits from a GnRH based primary assay can be tested for the presence of polynucleotide interacting compounds, particularly compounds that bind to duplex polynucleotide.

### Example 6B - GLP

GLP-1 (glucagon-like peptide-1) is a gut hormone which is released into the blood stream after feeding. Its main action is to stimulate insulin secretion through potentiating the insulinotropic action of glucose. The peptide is encoded in the glucagon gene and expressed mainly in the gut L cells. It exerts its actions through activating specific receptors of the seven transmembraneous domain-G-protein-coupled type with 463 amino acids. Its main signaling mechanism is activation of adenylate cyclase and formation of cyclic AMP. The peptide also increases the cytoplasmic concentration of Ca2 which is thought to be executed both through a Na (+)-dependent uptake of extracellular Ca2+ and through release of Ca2+ from intracellular Ca2+ stores. GLP-1 also inhibits glucagon secretion and inhibits gastric emptying and gastric acid and pancreatic exocrine secretion. Its integrated action on carbohydrate metabolism results in reduction of circulating glucose, and GLP-1 has therefore been suggested as a therapeutic alternative in diabetes. Finally, GLP-1 is also expressed in neurons in the hypothalamus, and may be involved in the regulation of feeding behavior, since it inhibits food intake [Ahren, *B., Bioessays,* **20**(8): 642-51 (8/1998)].

Using high-throughput technology, scientists can rapidly search for new antagonists and agonists, inhibitors and activators, of GLP. However, the primary assays fail to screen out "negative" leads, such as potential carcinogens and intercalating compounds. Using the ESI-MS technique described herein, a pool of primary hits from a GLP based primary assay can be tested for the presence of polynucleotide interacting compounds, particularly compounds that bind to duplex polynucleotide.

Other aspects, advantages, and modifications to the invention, described above in detail, will be readily apparent to those skilled in the art. For example, by using different types of dissociation techniques, including but not limited to CID, ion trap, IR-MPD, and competitive binding, one may identify not only the presence of interaction with the polynucleotide but also the class of the interaction, distinguishing intercalators from groove binders, major from minor groove binders. These alternate techniques can provide additional information regarding structural mapping and details.

The use of ESI-MS to identify single molecule:polynucleotide (double- or single-stranded) interactions is not limited to the exact conditions and parameters described above. In fact, ESI-FT-ICR-MS can be used screen hundreds of small molecules in the presence of multiple DNA and RNA targets in a single sample. High resolution of FT-ICR-MS allows for the mass identification of small molecules and targets. Also, the addition of a multi-plate autosampler can allow for the unattended analysis of thousands of compounds per day.

While the invention has been described in conjunction with the detailed description thereof, it is to be understood that the foregoing description is exemplary and explanatory in nature, and is intended to illustrate the invention and its preferred embodiments. Through routine experimentation, the artisan will recognize apparent modifications and variations that may be made without departing from the spirit of the invention. Thus, the invention is intended to be defined not by the above description, but by the following claims and their equivalents.

## Claims

1. A method of measuring the binding between one or more test compounds and a substrate mixture of components comprising double-stranded polynucleotide and its associated complementary single-strands in equilibrium, comprising the steps of:
(a) providing a solution comprising at least one test compound and one substrate mixture;
(b) converting said solution to a gaseous spray of ions by electrospray ionization;
(c) transferring the ions within said spray into a mass spectrometer;
(d) monitoring the non-covalent complexes formed between the substrate mixture components and said test compound; and
(e) assessing the results of step (d) to identify those test compounds having specific non-covalent interactivity with said substrate mixture components.

2. The method of claim 1 wherein said assessment at step (e) is used to distinguish false positive signals from true positive signals in a primary screen of test compounds.

3. The method of claim 1 wherein said assessment at step (e) is used to identify putative carcinogens.

4. The method of claim 2 wherein said primary screen comprises a biological target which associates with a polynucleotide and said test compound is screened for its biological activity with respect to said target.

5. The method of claim 4 wherein said biological activity comprises inhibition of a biological process associated with said target.

6. The method of claims 5 wherein said biological process is a catalytic reaction.

7. The method of claim 6 wherein said catalytic reaction is an enzymatic reaction.

8. The method of claim 7 wherein the enzyme of said enzymatic reaction is selected from the group consisting of kinases, transferases, helicases, endonucleases, exonucleases, proteases, peptidases, phosphotases, and polymerases.

9. The method of claims 7 wherein said enzyme is a viral enzyme.

10. The method of claims 8 wherein said enzyme is a viral enzyme.

11. The method of claim 9 wherein said viral enzyme is made by a virus selected from the group consisting of human immunodeficiency virus, human papilloma virus, respiratory syncytial virus, hepatitis A virus, hepatitis B virus, hepatitis C virus, cytomegalovirus, herpes simplex virus, herpes zoster virus, Norwalk virus, Ebola virus, Epstein-Barr virus, Hanta virus, rhinovirus and influenza virus.

12. The method of claims 9 wherein said viral enzyme is associated with a critical step in the life cycle of the virus making said enzyme.

13. The method of claims 11 wherein said viral enzyme is associated with a critical step in the life cycle of the virus making said enzyme.

14. The method of claim 12 wherein said critical step is replication of viral DNA, RNA, or the viral genome.

15. The method of claim 13 wherein said critical step is integration of the viral genome into the host genome.

16. The method of claim 9 wherein said virus making said viral enzyme is causally connected to a pathological condition in a host.

17. The method of claim 16 wherein said pathological condition is associated with disease.

18. The method of claim 16 wherein said host is an animal.

19. The method of claim 16 wherein said host is human.

20. The method of claim 9 wherein said viral enzyme is selected from the group consisting of HCV helicase, HCV polymerase, HIV integrase, and HIV protease.

21. The method of claim 1 wherein said mass spectrometer is selected from the group consisting of quadrupole mass spectrometers, quadrupole ion trap, and ion cyclotron resonance.

22. The method of claim 1 wherein said polynucleotide substrates comprise double-stranded and single-stranded DNA oligonucleotides.

23. The method of claim 1 wherein said polynucleotide substrates comprise double- and single-stranded RNA oligonucleotides.

24. The method of claim 3 wherein said carcinogen is a polynucleotide interactor.

25. The method of claim 24 wherein said polynucleotide interactor is a DNA or RNA interactor.

26. The method of claim 26 wherein said DNA interactor is selected from the group consisting of duplex DNA or RNA intercalators and groove binders.
